Europäisches Patentamt

European Patent Office

Office européen des brevets

(11) Veröffentlichungsnummer: **0 229 771 B1**

## (12) EUROPÄISCHE PATENTSCHRIFT

(45) Veröffentlichungstag der Patentschrift:
11.09.91 Patentblatt 91/37

(51) Int. Cl.$^5$: **A61L 25/00, A61K 6/08**

(21) Anmeldenummer: 86903199.7

(22) Anmeldetag: 03.07.86

(86) Internationale Anmeldenummer:
**PCT/CH86/00092**

(87) Internationale Veröffentlichungsnummer:
**WO 87/00058 15.01.87 Gazette 87/01**

(54) KNOCHENZEMENT.

(30) Priorität: 05.07.85 CH 2920/85

(43) Veröffentlichungstag der Anmeldung:
29.07.87 Patentblatt 87/31

(45) Bekanntmachung des Hinweises auf die
Patenterteilung:
11.09.91 Patentblatt 91/37

(84) Benannte Vertragsstaaten:
**AT BE CH DE FR GB IT LI LU NL SE**

(56) Entgegenhaltungen:
EP-A- 0 017 937
EP-A- 0 026 090
EP-A- 0 123 323

(56) Entgegenhaltungen:
CH-A- 629 517
DE-A- 1 921 869
FR-A- 2 297 887
US-A- 4 243 763
US-A- 4 404 327
Implantatwerkstoff von J.F.Osborn

(73) Patentinhaber: BIOCEM AG
Stockerstrasse 8
CH-8002 Zürich (CH)

(72) Erfinder: BIOCEM AG
Stockerstrasse 8
CH-8002 Zürich (CH)

(74) Vertreter: Lusuardi, Werther Giovanni, Dr.
Dr. Lusuardi AG, Kreuzbühlstrasse 8
CH-8008 Zürich (CH)

## Beschreibung

Die Erfindung betrifft einen Knochenzement, der durch Aktivierung einer oder Vermischung mehrerer Komponenten zur Aushärtung gebracht werden kann, mit einer flüssigen, polymerisierbaren Phase, einem Gehalt an Füllstoffpartikeln und einem Polymerisationsaktivator.

Die bisher bekannt gewordenen Knochenzemente wurden primär als Verankerungsmaterial für Hüftgelenkprothesen entwickelt und eingesetzt. Sie basieren ausnahmslos auf der Basis Methyl-methacrylat (MMA) als organischer Matrix, vereinzelt auch mit plastifizierenden Zusätzen, beispielsweise Butylmethacrylat. Bedingt durch diesen einfachen chemischen Aufbau ist nur eine lineare Vernetzung der organischen Matrix möglich, was, unter Berücksichtigung der allgemein ungenügenden Vermischungstechnik und der anwendungsbedingten Verunreinigung des Knochenzementes mit Körperflüssigkeiten, zu einer extrem schwachen physikalischen Festigkeit und geringen Langzeitstabilität des auspoylmerisierten Knochenzementes führt.

Als weiterer Nachteil herkömmlicher Knochenzemente kommt deren, aufgrund der hohen Monomertoxizität des verwendeten Methyl-methacrylats bedingte, Inkompatibilität mit dem Knochengewebe hinzu, was zusammen mit der ohnehin schlechten physikalischen Festigkeit zu hohen Lockerungsraten (bei Implantaten), Brüchen, Desintegration, Abstossung durch das Knochengewebe, Entzündungen und Nekrotisierung des Knochengewebes und anderen unerwünschten Schädigungen sowohl kurzfristiger als auch langfristiger Natur führt.

EP-A-0 123 323 offenbart Knochenzemente welche auf difunktionellen Acrylsäure und Methacrylsäureestern basieren und als Füllstoff Apatit enthalten können. Über die Natur des verwendeten Apatit wird keine Aussage getroffen.

Eine Beimengung von bioinertem Hydroxylapatit zu einem solchen konventionellen Knochenzement, wie beispielsweise in der EU-B1 0'006'414 offenbart, führte zu keiner Verbesserung der Kompatibilität, da die im Uebermass vorhandenen Noxen (Methylmethacrylat, toxische Polymerisationsbeschleuniger, Exotherme) dies verunmöglichen.

Aus diesem Grunde konnte bis heute kein konventioneller Knochenzement als eigentliches Knochenersatz- oder Knochenverbundmaterial verwendet werden und beruht die Operationstechnik im Frakturbereich nach wie vor auf der Verwendung von Drahtligaturen oder Miniplatten.

Es bestand daher die Aufgabe, einen Knochenzement zu finden, der einerseits eine reproduzierbar höhere, physikalische und chemische Festigkeit und Langzeitstabilität und anderseits eine echte Biokompatibilität mit dem Knochen- und Weichteilgewebe (z.B. Dura, Bindegewebe, Flimmerepithel) aufweist.

Die Erfindung löst die gestellte Aufgabe mit einem Knochenzement, der die Merkmale des Anspruchs 1 aufweist.

Die besonderen Vorteile des erfindungsgemässen Knochenzementes liegen darin, dass einerseits durch Verwendung von Diacrylaten oder Dimethacrylaten, bzw. von Präpolymeren davon, eine dreidimensionale Vernetzung des ausgehärteten Knochenzementes resultiert mit einer langfristig signifikant höheren, physikalischen Festigkeit und anderseits die Resorbierbarkeit der Füllstoffpartikel ein Einwachsen des Knochengewebes in den Knochenzement ermöglicht und keine bindegewebige Umscheidung ausgebildet wird.

Ueberraschenderweise ergaben die radiologischen und histologischen Untersuchungen einen bisher nie beobachteten, direkten Knochen-Knochenzement-Kontakt mit teilweiser Verzahnung ohne jedes Anhaltszeichen für eine Primärnekrose. Bei der Verwendung des erfindungsgemässen Knochenzementes in der Schädelchirurgie für die Ueberbrückung von Kalotten-, Dura mater-, Unterkieferknochen- und Gehörhinterwand-Defekten, bzw. Refixation der Vorderwand des Sinus frontalis traten keine entzündlichen Reaktionen der Weichgewebe auf. Auch bei der direkten Ueberkappung von Zahnpulpen (Pulpawundverband) ergab sich ein einwandfreier Defektverschluss mit teilweise kanalisiertem Dentin unter unmittelbarer Anlagerung, d.h. ohne nekrotische Zwischenschicht, der neu gebildeten Hartsubstanz an den Knochenzement.

Diese echte Biokompatibilität, wie sie bisher von keinem der bekannten Knochenzemente auch nur annähernd erreicht wurde, konnte durch Elimination des hochtoxischen, in der überwiegenden Anzahl der bekannten Knochenzemente verwendeten Dimethyl-p-toluidins (DMPT) noch verbessert werde. Als besonders geeignete Polymerisationsbeschleuniger, welche für die vorgesehene Applikation die beste Gewebeverträglichkeit aufweisen, erwiesen sich solche aromatische Amine, welche ein Molekulargewicht von mindestens 167, vorzugsweise mindestens 195 besitzen, beispielsweise Toluidin- oder Xylidin-Derivate, im speziellen das N,N-Bis-(2-hydroxyaethyl)-p-toluidin oder -xylidin.

Die Menge des zu verwendenden Polymerisationsbeschleunigers hängt u.a. von der gewünschten Verarbeitung- und Aushärtungszeit des Knochenzementes ab. Für einen schnellärtenden Knochenzement, wie er in der Schädelchirurgie und für kleiner Defekte bevorzugt wird, liegt der Gehalt an Beschleuniger typischerweise zwischen 0,7 und 1,8 Gew.%, vorzugsweise zwischen 1,00 und 1,25 Gew.%, bezogen auf das Gesamtgewicht der den Polymerisationsbeschleuniger enthaltenden Komponente. Diese Gehaltsbereiche können variieren, falls als Polymerisationsstarter statt Benzoylperoxid ein anderer Katalysator verwendet wird, der Füllstoffanteil der Komponenten variiert wird oder den Polymerisationsvorgang beeinflussende Additive zugesetzt werden.

Die im erfindungsgemässen Knochenzement verwendeten Füllstoffpartikel können unterschiedliche Zusammensetzungen und geometrische Dimensionen aufweisen, welche dem spezifischen Anwendungszweck anzupassen sind. Die Verwendung von Apatit als Füllstoff in Form des penta-Calciumhydroxidtriphosphats oder des deka-Calciumdihydroxidhexaphophats ergab eine gute Verarbeitbarkeit und ein gutes Fliessvermögen der vermischten Knochenzementmasse. Durch seine grosse Aehnlichkeit mit dem natürlichen Knochenmaterial ergab sich auch eine ausgezeichnete Verträglichkeit.

Entgegen den in der Literatur vorgebrachten Bedenken bei der Verwendung poröser Apatitteilchen hat sich beim erfindungsgemässen Knochenzement ein Porenvolumen der Füllstoffpartikel von mindestens 0,2 ml/g, vorzugsweise von 0,3 ml/g überraschenderweise als besonders günstig für dessen physikalische Eigenschaften erwiesen.

Die zusätzliche Verwendung von biokeramischen Materialien und Gläsern in Form von Splittern oder Kugeln ergab eine schnellere Resorption mit zusätzlichen Verankerungsstellen für den wachsenden Knochen. Der Gehalt an biokeramischem Material oder Glas beträgt vorzugsweise höchstens 30 Gew.% bezogen auf die gesamte Menge des Füllstoffes. Zwei besonders geeignete biokeramische Materialien weisen folgende Zusammensetzungen auf:

|  | Biokeramik 1 (Gew.%) | Biokeramik 2 (Gew.%) |
|---|---|---|
| Siliziumdioxid | 42,4 | 40,0 |
| Calciumoxid | 22,0 | 24,5 |
| Phosphorpentoxid | 11,2 | 6,0 |
| Natriumoxid | 24,4 | 24,5 |
| Berylliumoxid | ---- | 5,0 |

Diese Zusammensetzungen werden bei ca. 1450°C erschmolzen, in Formen gegossen und abgekühlt. Anschliessend wird bei 700-750°C während etwa 2 Stunden thermisch behandelt um Misch-Kristallphasen vom Typ Larnit (ca. 23 Vol.%) und Devritit (ca. 68 Vol.%) zu erhalten. Als besonders bioinerte Biokeramik hat sich eine solche herausgestellt, welcher zusätzlich 5-10 Gew.% Lantanoxid ($La_2O_3$) zugesetzt wurde und welche die Bildung einer Apatitphase vom Typ $(Ca,La)_5(PO_4)_3$ ermöglicht.

Eine weitere bedeutende Verbesserung des erfindungsgemässen Knochenzementes ergab sich durch zusätzliche Beimischung von Calciumhydroxid, resp. Calciumoxid. Der damit angereicherte Knochenzement kann durch seine erhöhte Adhäsionsfähigkeit und Benetzbarkeit des Knochens mit Leichtigkeit am Knochen appliziert werden ohne den bekannten Retraktionseffekt. Das mit Calciumhydroxidzusatz erzeugte alkalische Milieu, mit einem kurzfristig erreichten pH-Wert von 10, wirkte zudem entzündungshemmend. Das Calciumhydroxid kann entweder einer der beiden redoxhärtenden Komponenten des Knochenzementes beigemischt oder als dritte Komponente, zweckmässigerweise in Form einer Paste auf Epoxidharz- oder Weichmacherbasis, eingesetzt werden. Vorteilhafterweise sind bei einer Beimischung von Calciumhydroxid nur solche Monomeren in der betreffenden Komponente zu verwenden, die keine freien Hydroxylgruppen aufweisen, um eine unerwünschte Hydrolyse des Monomers zu vermeiden. Als für diesen Zweck geeignete Harze haben sich vorallem Dimethacrylate mit Urethanbausteinen herausgestellt, wie beispielsweise das 1,6-Bis[methacryloxy-ethoxy-carboxamido]-2,2,4(2,4,4)-trimethylhexan.

Der Gehalt an Calciumhydroxid beträgt vorzugsweise zwischen 10-25 Gew.% bezogen auf das Gesamtgewicht der Knochenzementmasse.

Als für den erfindungsgemässen Knochenzement geeignete Diacrylate oder Dimethacrylate haben sich insbesondere höhermolekulare Dimethacrylate, vorzugsweise vom Bisphenol A Typ erwiesen. Speziell geeignet ist das 2,2-Bis[4-(2-hydroxy-3-methacroyloxy-propoxy)phenyl]propan, das zweckmässigerweise mit einem niedrigmolekulareren, dünnflüssigen Diacrylat oder Dimethacrylat verdünnt wird. Der Gehalt an Diacrylat, bzw.

Dimethacrylat beträgt vorteilhafterweise zwischen 30 und 50 Gew.%, vorzugsweise zwischen 35 und 45 Gew.% der einzelnen mit Füllstoff angereicherten Komponenten, mit einem Anteil von 30-50 Gew.% höhermolekularen Dimethacrylaten, vorzugsweise vom Bisphenol A Typ.

Zur Unterdrückung einer durch den Luftsauerstoff bewirkten Polymerisationsinhibierung an der Oberfläche des aushärtenden Knochenzementes hat sich die zusätzliche Beimischung von 1-5 Gew.% 2,2-Bis[4-methacroyloxy-phenyl]propan als zweckmässig erwiesen.

Für Anwendungen bei denen grössere Mengen an Knochenzement zum Auspolymerisieren gebracht werden müssen, beispielsweise zur Implantation von Hüftgelenkprothesen, und demzufolge eine erhebliche Exotherme auftritt, hat sich überraschenderweise gezeigt, dass die Wärmeentwicklung durch zusätzliche Verwendung von 0,1 bis 2,0 Gew.% (bezogen auf die Menge der flüssigen Phase) eines Menthadiens, vorzugsweise von 1-Methyl-4-isopropyl-cyclohexadien-(1.8) drastisch reduzieren lässt bei gleichbleibender Aushärtungszeit.

Den eingesetzten Diacrylaten, bzw. Dimethacrylaten können die üblichen Stabilisatoren (z.B. 2,6-Ditert.butyl-phenol), UV-Absorber, Thixotropiermittel (mikrofeine Kieselsäure) und andere geeignete Additive zugesetzt werden. Für Anwendungen bei denen eine spätere röntgenologische Kontrolle wichtig ist, können 5-10 Gew.% eines Röntgenopakmittels (z.B. Zirkondioxid) zugesetzt werden.

Für bestimmte Anwendungszwecke kann es vorteilhaft sein, dem erfindungsgemässen Knochenzement Weichmacher zur Verringerung der Sprödigkeit zuzusetzen. Gut geeignet sind besonders solche auf Basis von hochmolekularen Polyurethanen, Polycarbonaten, Polyestern und Polyäthern, beispielsweise dem Nonylphenolpolyglykol-ätheracetat.

Günstige Darbietungsformen für ein mit Peroxid und Beschleuniger aktiviertes System sind die 2-Pasten-Form (Radikalstarter in der einen Paste / Beschleuniger in der anderen Paste) und das Pulver-Flüssigkeit-System (Radikalstarter im Pulver / Beschleuniger in der Flüssigkeit), wobei die Aushärtung durch Vermischen beider Pasten, bzw. durch Mischen des Pulvers mit der Flüssigkeit eingeleitet wird.

Es ist jedoch auch möglich, einphasige Knochenzemente herzustellen, die unter Einwirkung von elektromagnetischer Strahlung, z.B. UV-Licht, sichtbares Licht oder Laserlicht polymerisieren und dann einen Photopolymerisationsinitiator (z.B Benzoin und dessen Derivate) und gegebenenfalls auch einen Polymerisationsbeschleuniger dafür enthalten.

Der erfindungsgemässe Knochenzement eignet sich insbesondere für die folgenden Applikationen:

### 1. In der Schädel-/Gesichtschirurgie:

1.1. Zur Auffüllung von Knochendefekten im Schädelbereich und zur Knochensplitterfixation.

1.2. Zur Auffüllung kleinerer Knochendefekte bei anderen Knochen, z.B. als Ersatz für Schrauben und Ligaturen.

1.3. Zur Reposition von osteotomierten, autogenen Knochentransplantaten, bzw. zur Fixation von Alloimplantaten am Knochen.

### 2. Im Dentalbereich:

2.1. Als Primärfixation zur Stabilisierung von oralen Implantaten.
2.2. Als Ersatzdentin bei der Eröffnung der vitalen Pulpa (Pulpa-Ueberkappung).
2.3. Als Wurzelfüllmaterial.
2.4. Zur retrograden Wurzelspitzenfüllung mit Knochendefektauffüllung apikal.
2.5. Zur Apikalverankerung lockerer Zähne.

### 3. In der HNO-Chirurgie:

3.1. Zur Rekonstruktion der hinteren Gehörgangwand bei Radikaloperationen wegen Mittelohrcholesteatom.
3.2. Zur Verkleinerung von Radikalhöhlen.
3.3. Zur Gehörknöchelchenrekonstruktion, vor allem nach Frakturen.

### 4. In der Orthopädie:

4.1. Zur Zementierung von künstlichen Gelenkkomponenten, insbesondere Hüft- und Kniegelenken.

Nachfolgend wird an Hand von beispielen die Erfindung näher erläutert. Die Tabellen zeigen dabei die chemische und gewichtsmässige Zusammensetzung der verschiedenen Komponenten.

## Tabelle 1

| Zusammensetzung | Komponente 1 (Gew.-%) | Komponente 2 (Gew.-%) | Komponente 3 (Gew.-%) | Komp. 4 (Gew.-%) | Komp. 5 (Gew.%) | Komp. 6 (Gew.%) | Komp. 7 (Gew.%) |
|---|---|---|---|---|---|---|---|
| 2,2-Bis[4-(2-hydroxy-3-methacryloxy-propoxy)-phenyl]propan | 14,0 | 14,0 | | 19,4 | 19,4 | 23,57 | 25,39 |
| 2,2-Bis[4-methacryloxy-phenyl]propan | 2,0 | 2,0 | | 2,8 | 2,8 | | |
| Triaethylenglykol-dimethacrylat | 22,4 | 22,4 | | 31,1 | 31,1 | 23,57 | 25,39 |
| Benzoylperoxid | 1,61 | | | 2,2 | | | 0,92 |
| N,N-Bis-(2-hydroxyaethyl)-p-toluidin | | 1,125 | | | 1,6 | 0,48 | |
| $Ca_5(PO_4)_3OH$ | 59,99 | 60,475 | | 44,36 | 45,1 | 26,19 | 48,284 |
| Calciumhydroxid | | | 100 | | | 26,185 | |
| 1,6-Bis[methacryloxy-ethoxy-carboxamido]2,2,4(2,4,4)-trimethylhexan | | | | | | | |
| Mikrofeine Kieselsäure | | | | | | | |
| Stabilisator | | | | 0,14 | | 0,005 | 0,016 |
| UV-Absorber | | | | | | | |
| Weichmacher | | | | | | | |
| Zirkondioxid | | | | | | | |

Tabelle 2

| Zusammensetzung (Gew.%) | Komp. 8 | Komp. 9 | Komp.10 | Komp. 11 | Komp. 12 | Komp.13 | Komp.14 | Komp. 15 | Komp.16 |
|---|---|---|---|---|---|---|---|---|---|
| 2,2-Bis[4-(2-hydroxy-3-methacroyloxy-propoxy)-phenyl]propan | | 24,27 | | 28,63 | .29,12 | 21,75 | 21,52 | 7,23 | 7,16 |
| 2,2-Bis[4-methacroyloxy-phenyl]propan | | | | | | | | | |
| Triaethylenglykol-dimethacrylat | 25,514 | | 23,66 | 28,63 | 29,12 | 21,75 | 21,52 | | |
| Benzoylperoxid | | | | 1,03 | | 0,78 | | | 0,99 |
| N,N-Bis-(2-hydroxyaethyl)-p-toluidin | | | | | 0,59 | | 0,43 | 0,7 | |
| Ca$_5$(PO$_4$)$_3$OH | 35,9 | 36,59 | 38,1 | 41,53 | 41,164 | 55,707 | 56,526 | | |
| Calciumhydroxid | 12,82 | 14,63 | 14,3 | | | | | | |
| 1,6-Bis[methacryloxy-ethoxy-carboxamido]2,2,4(2,4,4)-trimethylhexan | 25,51 | 24,27 | 23,7 | | | | | | |
| Mikrofeine Kieselsäure | | | | | | | | 7,0 | 7,0 |
| Stabilisator | | | | 0,018 | 0,006 | 0,013 | 0,004 | | 0,04 |
| UV-Absorber | 0,256 | 0,24 | 0,24 | | | | | | |
| Weichmacher | | | | | | | | 55,07 | 54,81 |
| Zirkondioxid | | | | | | | | 30,0 | 30,0 |

EP 0 229 771 B1

Beispiel 1:

Von den beiden getrennt in Spritzen aufbewahrten Komponenten 1 und 2 wurden je 0,3 g während 30 Sekunden miteinander vermischt und zur Auffüllung eines Tibiadefektes bei 37°C verwendet. Die Aushärtungszeit betrug 2,75 Minuten und die dabei erreichte Höchsttemperatur 44°C.
Die Probe war ausgezeichnet ausgehärtet, insbesondere auch an deren Oberfläche.

Beispiel 2:

Je 0,3 g der Komponenten 1 und 2 wurden mit 0,06 g der Komponente 3 vermischt und analog zu Beispiel 1 appliziert. Man erhielt neben einer verbesserten Handhabung der Knochenzementmasse die gleichen vorteilhaften Resultate wie in Beispiel 1.

Beispiel 3:

Je 0,2 g der Komponenten 4 und 5 wurden während 30 Sekunden vermischt und zur Auffüllung eines Defektes an einem Modellknochen bei 23°C verwendet. Die Aushärtung erfolgte nach ca. 3 Minuten mit einer Höchsttemperatur von 28°C.
Bei Verwendung von je 0,3 g der beiden Komponenten bei 37°C erfolgte die Aushärtung innerhalb von ca. 2,75 Minuten mit einer Maximaltemperatur von 44°C.
Die Oberflächen der ausgehärteten Proben waren alle gut auspolymerisiert.
Dieses 2-Komponentensystem wurde als Ueberkappungsmaterial verwendet und zeigte auch histologisch gute Ergebnisse.

Beispiel 4:

Gleiche Mengen der Komponenten 6 und 7 wurden während 60 Sekunden vermischt und für eine direkte Pulpaüberkappung verwendet. Es traten postoperativ keine Komplikationen auf. Die histologische Aufarbeitung ergab ebenfalls ausgezeichnete Ergebnisse.

Beispiel 5:

Je eine Probe der Einkomponentensysteme 8,9 und 10 wurden zur direkten Pulpaüberkappung verwendet und mit einer UV-Lampe innerhalb von 20 bis 40 Sekunden an Ort und Stelle ausgehärtet. Die Aushärtung erfolgte auch im UV-Lichtschatten, so dass ein durchgehend auspolymerisiertes Material resultierte.
Die Laborprüfung des ausgehärteten Materials ergab einen pH-Wert zwischen 9,2 und 11,0.

Beispiel 6:

Gleiche Mengen (0,225 g) der Komponenten 11 und 12 wurden während 30 Sekunden miteinander vermischt und zur Rekonstruktion einer Gehörhinterwand verwendet. Sowohl konsistenzmässig (eher dünnflüssig) als auch von der Aushärtungszeit (ca. 2,5 min) ist das Material für diesen Zweck gut geeignet.

Beispiel 7:

Gleiche Mengen (0,25 g) der Komponenten 13 und 14 wurden während 30 Sekunden miteinander vermischt und zur Defektauffüllung bei Wurzelspitzenresektionen verwendet. Im Vergleich zum Material gemäss Beispiel 6 war die Konsistenz eher dick, die exotherme Reaktion geringer (ca. 50°C) und die Aushärtungszeit leicht kürzer (2 min).

Beispiel 8:

Gleiche Mengen (0,1 g) der Komponenten 15 und 16 wurden während 60 Sekunden vermischt und als Wurzelfüllungsmaterial verwendet. Die Aushärtungszeit betrug 15 Minuten.

# EP 0 229 771 B1

## Patentansprüche

1. Knochenzement der durch Aktivierung einer oder Vermischung mehrerer Komponenten zur Aushärtung gebracht wird, mit einer flüssigen, polymerisierbaren Phase, welche mindestens teilweise auf einem Diacrylat oder Diruethacrylat basiert, einem Gehalt an Füllstoffpartikeln und einem Polymerisationsaktivator, dadurch gekennzeichnet, dass Hydroxylapatitpartikeln besteht.

2. Knochenzement nach Anspruch 1, dadurch gekennzeichnet, dass eine Komponente als Polymerisationsbeschleuniger ein aromatisches Amin mit einem Molekulargewicht von mindestens 167, vorzugsweise mindestens 195, beispielsweise ein Toluidin- oder Xylidinderivat enthält.

3. Knochenzement nach Anspruch 1 oder 2, dadurch gekennzeichnet, dass die porösen Hydroxylapatitpartikel in Form des penta-Calciumhydroxidtriphosphats oder des deka-Calciumdihydroxidhexaphosphats vorliegen.

4. Knochenzement nach einem der Ansprüche 1 bis 3, dadurch gekennzeichnet, dass die porösen Hydroxylapatitpartikel ein Porenvolumen von mindestens 0,2 ml/g, vorzugsweise von mindestens 0,3 ml/g, aufweisen.

5. Knochenzement nach einem der Ansprüche 1 bis 4, dadurch gekennzeichnet, dass die porösen Hydroxylapatitpartikel mindestens teilweise aus vorzugsweise eine Apatitphase enthaltenden biokeramischem Material oder Biogläsern bestehen, vorzugsweise in einer Menge von höchstens 30 Gew.% bezogen auf das gesamte Füllstoffgewicht.

6. Knochenzement nach einem der Ansprüche 1 bis 5, dadurch gekennzeichnet, dass mindestens 5 Gew.%, vorzugsweise mehr als 10 Gew.% der porösen Hydroxylapatitpartikel einen Durchmesser von weniger als 35µ, vorzugsweise von weniger als 25µ aufweist.

7. Knochenzement nach einem der Ansprüche 1 bis 6, dadurch gekennzeichnet, dass dieser zusätzlich einen Anteil an feinkörnigen Kunststoffperlen, vorzugsweise aus einem Methylmethacrylat/Methylacrylat/Copolymer, enthält, vorzugsweise in einer Menge von 60 bis 70 Gew.% bezogen auf das Gesamtgewicht der ausgehärteten Knochenzementmasse.

8. Knochenzement nach einem der Ansprüche 1 bis 7, dadurch gekennzeichnet, dass als selbständige Komponente oder als Zumischung zu einer oder mehreren der Knochenzement-Komponenten Calciumhydroxid oder Calciumoxid verwendet wird.

9. Knochenzement nach einem der Ansprüche 1 bis 8, dadurch gekennzeichnet, dass die flüssige Phase keine hydroxylgruppenhaltige Monomeren enthält.

10. Knochenzement nach einem der Ansprüche 1 bis 9, dadurch gekennzeichnet, dass die flüssige Phase ein Bisphenol A Derivat enthält, beispielsweise das 2,2-Bis[4-(2-hydroxy-3- methacroyloxy-propoxy)phenyl]propan, vorzugsweise verdünnt mit einem niedrigmolekulareren, dünnflüssigeren Diacrylat oder Dimethacrylat.

11. Knochenzement nach einem der Ansprüche 1 bis 10, dadurch gekennzeichnet, dass die flüssige Phase zusätzlich ein Menthadien-Derivat, vorzugsweise 1-Methyl-4-isopropyl-cyclohexadien-(1.8), enthält.

12. Knochenzement nach einem der Ansprüche 1 bis 11, dadurch gekennzeichnet, dass die flüssige Phase mindestens teilweise ein Urethandimethacrylat, vorzugsweise das 1,6-Bis[methacryloxy-ethoxy-carboxamido]-2,2,4(2,4,4)-trimethylhexan, enthält.

13. Knochenzement nach einem der Ansprüche 1 bis 12, dadurch gekennzeichnet, dass die flüssige Phase zusätzlich einen Gehalt an 2,2-Bis[4-methacroyloxy-phenyl]propan aufweist.

## Claims

1. Bone cement to be hardened by activating one or mixing several components, with a liquid, polymerizable phase which is based at least partially on a diacrylate or dimethacrylate, a content of filler particles and a polymerisation activator, characterised in that at least a part of the filler particles consists of porous hydroxyapatite particles.

2. Bone cement according to claim 1, characterised in that one component contains an aromatic amine with a molecular weight of at least 167, preferably at least 195, for example a derivative of toluidine or xylidine, as a polymerisation accelerator.

3. Bone cement according to claim 1 or 2, characterised in that the porous hydroxyapatite particles are present in form of the penta-calciumhydroxytriphosphate or the deca-calcium-dihydroxyhexaphosphate.

4. Bone cement according to one of the claims 1 to 3, characterised in that the porous hydroxyapatite particles have a pore volume of at least 0.2 ml/g, preferably of at least 0.3 ml/g.

5. Bone cement according to one of the claims 1 to 4, characterised in that the porous hydroxyapatite par-

8

ticles consist at least partially of bioceramic material or bioglasses, preferably containing an apatite phase, in an amount of preferably maximum 30 percent in weight with regard to the full weight of the fillers.

6. Bone cement according to one of the claims 1 to 5, characterised in that at least 5 percent in weight, preferably more than 10 percent in weight of the porous hydroxyapatite particles have a diameter of less than 35μ, preferably less than 25μ.

7. Bone cement according to one of the claims 1 to 6, characterised in that it additionally contains a portion of fine grained plastic beads, preferably from a methylmethacrylate/ methylacrylate/copolymer, preferably in an amount of 60 to 70 percent in weight with regard to the total weight of the hardened bone cement mass.

8. Bone cement according to one of the claims 1 to 7, characterised in that calciumhydroxide or calciumoxide is used as an independent component or as an admixture to one or several of the bone cement components.

9. Bone cement according to one of the claims 1 to 8, characterised in that the liquid phase does not contain monomers with hydroxy groups.

10. Bone cement according to one of the claims 1 to 9, characterised in that the liquid phase contains a bisphenol A derivative, for example the 2,2-bis[4-(2-hydroxy-3-methacroyloxy-propoxy)phenyl]propane, preferably diluted with a low molecular, lower viscosity diacrylate or dimethacrylate.

11. Bone cement according to one of the claims 1 to 10, characterised in that the liquid phase contains additionally a derivative of menthadiene, preferably 1-methyl-4-isopropyl-cyclohexadiene-(1.8).

12. Bone cement according to one of the claims 1 to 11, characterised in that the liquid phase contains at least partially a urethanedimethacrylate, preferably the 1,6-bis-[methacroyloxy-ethoxy-carboxamido]-2,2,4(2,4,4)-trimethylhexane.

13. Bone cement according to one of the claims 1 to 12, characterised in that the liquid phase contains additionally an amount of 2,2-bis[4-methacroyloxy-phenyl]propane.

## Revendications

1. Ciment osseux à faire durcir par l'activation d'un ou le mélange de plusieurs composants, avec une phase polymérisable, liquide, qui est basée, au moins en partie, sur un diacrylate ou un diméthacrylate, une teneur en particules d'une charge et un activateur de polymérisation, caractérisé en ce qu'au moins une part des particules de la charge se composent de particules d'hydroxylapatite poreuses.

2. Ciment osseux suivant la revendication 1, caractérisé en ce qu'un composant contient, à titre d'accélérateur de polymérisation, une amine aromatique qui possède un poids moléculaire d'au moins 167, de préférence d'au moins 195, par exemple un dérivé de xylidine ou de toluidine.

3. Ciment osseux suivant la revendication 1 ou 2, caractérisé en ce que les particules d'hydroxylapatite poreuses se présentent sous la forme d'hydroxytriphosphate pentacalcique ou de dihydroxyhexaphosphate décacalcique.

4. Ciment osseux suivant l'une des revendications 1 à 3, caractérisé en ce que les particules d'hydroxylapatite poreuses présentent un volume des pores d'au moins 0,2 ml/g, de préférence d'au moins 0,3 ml/g.

5. Ciment osseux suivant l'une des revendications 1 à 4, caractérisé en ce que les particules d'hydroxylapatite poreuse se composent, au moins en partie, de bioverres ou d'un matériau biocéramique, de préférence en une proportion maximale de 30% en poids par rapport au poids total de la charge.

6. Ciment osseux suivant l'une des revendications 1 à 5, caractérisé en ce qu'au moins 5% en poids, de préférence plus de 10% en poids, des particules d'hydroxylapatite poreuses présentent un diamètre inférieur à 35 μ, de préférence inférieur à 25 μ.

7. Ciment osseux suivant l'une des revendications 1 à 6, caractérisé en ce qu'il contient, complémentairement une fraction de perles d'une matière plastique à fins grains, de préférence en un copolymère de méthacrylate de méthyle et d'acrylate de méthyle, de préférence en une proportion de 60 à 70% en poids, par rapport au poids total de la masse du ciment osseux durci.

8. Ciment osseux suivant l'une des revendications 1 à è, caractérisé en ce que l'on utilise de l'hydroxyde de calcium ou de l'oxyde de calcium à titre de composant indépendant ou à titre d'additif à un ou plusieurs des composants du ciment osseux.

9. Ciment osseux suivant l'une des revendications 1 à 8, caractérisé en ce que la phase liquide ne contient pas de monomères à radicaux hydroxyle.

10. Ciment osseux suivant l'une des revendications 1 à 9, caractérisé en ce que la phase liquide contient un dérivé du bisphénol A, par exemple le 2,2-bis[4-(2-hydroxy-3-méthacroyloxy-propoxy)phényl]propane, de préférence dilué avec un diméthacrylate ou un diacrylate de faible poids moléculaire de plus forte fluidité.

11. Ciment osseux suivant l'une des revendications 1 à 10, caractérisé en ce que la phase liquide contient complémentairement un dérivé du menthadiène, de préférence le 1-méthyl-4-isopropylcyclohexadiène-(1,8).

12. Ciment osseux suivant l'une des revendications 1 à 11, caractérisé en ce que la phase liquide contient au moins un diméthacrylate d'uréthanne, de préférence le 1,6-bis-[méthacroyloxy-éthoxy-carboxamido]-2,2,4(2,4,4)-triméthylhexane.

13. Ciment osseux suivant l'une des revendication 1 à 12, caractérisé en ce que la phase liquide contient complémentairement une certaine proportion de 2,2-bis-[4-méthacroyloxy-phényl]propane.